(19) Europäisches Patentamt
European Patent Office
Office européen des brevets

(11) Publication number: **0 121 502**
**B1**

(12) **EUROPEAN PATENT SPECIFICATION**

(45) Date of publication of patent specification: **08.04.87**

(51) Int. Cl.[4]: **C 07 D 499/00,** A 61 K 31/43
// C07D205/08

(21) Application number: **84810164.8**

(22) Date of filing: **03.04.84**

(54) **Novel penem compounds, pharmaceutical compositions containing them, processes for their preparation.**

(30) Priority: **04.04.83 US 481551**

(43) Date of publication of application:
**10.10.84 Bulletin 84/41**

(45) Publication of the grant of the patent:
**08.04.87 Bulletin 87/15**

(84) Designated Contracting States:
**AT BE CH DE FR IT LI LU NL SE**

(56) References cited:
**EP-A-0 002 210**
**EP-A-0 013 662**
**GB-A-2 097 786**

**CHEMICAL ABSTRACTS, vol. 99, no. 23, 5th December 1983, page 730, no. 194712w, Columbus, Ohio, US; & JP - A - 58 43 978 (SANKYO CO., LTD.) 14-03-1983**

**The file contains technical information submitted after the application was filed and not included in this specification**

(73) Proprietor: **SCHERING CORPORATION**
**2000 Galloping Hill Road**
**Kenilworth, New Jersey 07033 (US)**

(72) Inventor: **Girijavallabhan, Viyyoor Moopil**
**10 Maplewood Drive**
**Parsippany New Jersey 07054 (US)**
Inventor: **Ganguly, Ashit Kumar**
**96 Cooper Avenue**
**Upper Montclair New Jersey 07043 (US)**
Inventor: **McCombie, Stuart Walter**
**28 Harford Place**
**Caldwell New Jersey 07006 (US)**

(74) Representative: **Ritter, Stephen David et al**
**Mathys & Squire 10 Fleet Street**
**London EC4Y 1AY (GB)**

**EP 0 121 502 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to 5R,6S,8R-6-(1-hydroxyethyl)-2-(2-carbamoyloxyethylthio)-penem-3-carboxylic acid and its pharmaceutically acceptable salts and metabolizable esters.

As used herein, "pharmaceutically acceptable salts" preferably means alkali metal salts such as sodium and potassium salts; alkaline earth metal salts such as calcium, magnesium and aluminium salts, amine salts formed from a wide variety of suitable organic amines, i.e., aliphatic, cycloaliphatic, (cycloaliphatic)aliphatic or araliphatic primary, secondary or tertiary mono-, di- or polyamines, or heterocyclic bases, e.g., salts derived from triethylamine, 2-hydroxyethylamine, di-(2-hydroxyethyl)amine, tri-(α-hydroxyethyl)amine, 4-aminobenzoic acid-2-diethylaminoethyl ester, 1-ethylpiperidine, bicyclohexylamine, N,N'dibenzylethylenediamine, pyridine, collidine, quinoline, procaine, dibenzylamine, 1-ephenamine (β-methylamino-α-phenyl-benzene ethanol) and N-alkylpiperidine.

"Pharmaceutically acceptable metabolizable esters" means physiologically cleavable esters, i.e., those esters known in the penicillin, cephalosporin and penem arts to be easily cleaved within the body of the parent acid. Examples of such esters are indanyl, phthalidyl, methoxymethyl, glycyloxymethyl, phenylglycyloxymethyl, thienylglycyloxymethyl, acetoxymethyl and pivaloyloxymethyl.

Our published EP—A—0013662 discloses a class of penems substituted in the 2-position by certain alkylthio and substituted alklythio groups. Penems substituted in the 2-position by sulfur-containing substituents are also disclosed in Japanese Patent Application No. 56—142739 (JP—A—58—43978), whereas GB—A—2097786 discloses a penem compound substituted in the 2-position by a 2-carbamoyloxymethyl group. We have found that the compounds of the present invention possess unexpectedly superior activity against gram-negative and gram-positive bacteria compared with penem compounds named hitherto. In addition the compounds of this invention exhibit low protein binding and their metabolites have little or no unpleasant odour.

When tested in standardized microbiological assays, the compounds of this invention are active against such gram-positive organisms as *Staphylococcus epidermis* and *Baccillus subtilis*, and such gram-negative organisms as *E. coli* and *Salmonella*, at test levels of below 2.0 micrograms/ml. Additonally, they show activity against organisms which produce beta-lactamases, e.g., penicillinase and cephalosporinase, indicating a resistance against these enzymes. For instance, the sodium salt is active against *E. coli* 74081501 (a beta-lactamase producing organism) at test level of 0.1250 microgram/ml.

As antibacterial agents, the compounds of this invention are conventionally formulated for oral, parenteral, topical and transdermal use, preferably parenteral. Thus, this invention includes within its scope pharmaceutical compositions comprising the compounds of this invention, in admixture with a pharmaceutically acceptable carrier or excipient.

In the foregoing compositions the compounds of this invention can be used as the sole active antibacterial agent or in combination with other antibacterial agents, enzyme inhibitors and/or absorption enhancers.

For the preferred composition, for parenteral administration, a compound in accordance with the invention may be formulated into solution or suspension. Typical topical formulations are those such as lotions, creams, ointments, sprays, and mechanical delivery devices, e.g., transdermal. For oral administration the compounds of this invention are typically formulated in the form of tablets, capsules, elixirs or the like.

As well as pharmaceutically acceptable carriers, other non-toxic compatible fillers, binders, disintegrants and lubricants commonly used in pharmaceutical formulations may be included. The compositions may also contain preservatives, thickening, suspending, dispensing, emulsifying, wetting, stabilizing and buffering agents.

The dosage of a compound in accordance with this invention which is administered, will be in accordance with the judgement of the attending clinician and depend upon a variety of factors, i.e. the age and weight of the individual being treated, the mode of administration and the type and severity of the bacterial infection being prevented or reduced. Typically, the dosage administered per day will be in the range of from about 25 to 160 mg/kg and preferably from about 50 to 100 mg/kg in divided dosages. Typically, the dosage will be administered in dosage units containing convenient amounts, for example, 0.250, 0.500, 1 or 2 gms of active ingredient combined with a suitable physiologically acceptable carrier or diluent.

According to a further aspect of the invention, a process for preparing compounds of formula I and their pharmaceutically acceptable salts and ester is characterized by

A) reaction of a compound of the general formula

OPg; S; $SCH_2CH_2OH$; N; O; COOPr — I

wherein Pg is a hydroxy protecting group and Pr is a carboxy protecting group, with an alkali metal cyanate in the presence of trifluoroacetic acid or trichloroacetic acid;

B) reaction of a compound of the general formula

II

in which R is an organic radical different from carbamoyloxyethyl and any functional groups are protected if necessary or desired, with a compound of the general formula

$$HS\ CH_2CH_2O{-}\overset{\displaystyle O}{\overset{\|}{C}}{-}NH_2 \qquad III$$

or a reactive derivative thereof;

C) reaction of a compound of the general formula

IVa ⇌ IVb

in which any functional groups are protected if necessary or desired, with a compound having the general formula

$$L{-}CH_2CH_2O{-}\overset{\displaystyle O}{\overset{\|}{C}}{-}NH_2 \qquad V$$

in which L is a leaving group,

D) reaction of a compound of the general formula

VI

in which any functional groups are protected if necessary or desired, with a trivalent organophosphorus compound

the process A, B, C, or D being followed by one or more of the following operations, if necessary or desired;

(i) deprotection of one or more protected groups;

(ii) separation of the (5R,6S,8R) stereoisomer;

(iii) conversion of a free acid to a pharmaceutically acceptable salt or metabolisable ester;

(iv) conversion of a salt or ester to a free acid;

(v) transesterifying an ester or converting an ester into a pharmaceutically acceptable salt;

(vi) converting a salt into another salt or an ester.

*Process A* is generally carried out in an organic solvent, e.g. benzene or methylene chloride at a temperature of 0°C to 35°C, preferably at about 25°C, e.g. room temperature. The preferred solvent is methylene chloride. The preferred alkali metal cyanates are potassium and sodium cyanate. The preferred acid is trifluoroacetic acid.

*Process B* involving sulfoxide replacement is usually carried out in an inert solvent, for example dichloromethane or tetrahydrofuran. The reaction is usually carried out under cooling e.g. at −70°C to 10°C, preferably 0°C to 5°C.

When the thiol compound is itself used the reaction is generally carried out in the presence of a base, e.g. an organic base such as diisopropylethylamine or triethylamine, or an inorganic base. Alternatively a reactive derivative of the thiol, for example an alkali metal salt preferably a sodium or potassium salt may be used.

*Process C* is generally carried out in a solvent e.g. an organic solvent possibly containing water e.g. acetonitrile and water, or an anhydrous organic solvent e.g. tetrahydrofuran, ethylther or dioxan. Generally the reaction will be carried out in the presence of a base which may be an organic base e.g. triethylamine, or more usually an inorganic base such as sodium bicarbonate. Reaction temperatures are typically from about −10°C to 45°C e.g. 10°C to 45°C with room temperature (about 25°C) being preferred.

Preferred leaving groups L are halogen e.g. iodo, trifluoroacetate and triphenylphosphonium-oxy.

*Process D* involving cyclisation of the compound of formula VI is usually carried out analogously to the process described in European Patent Application Publication No. 58317. Thus it is usually carried out in an inert solvent, for example an aromatic hydrocarbon e.g. toluene, benzene, aliphatic ethers, e.g. diethylether and dipropylether, cyclic ethers, e.g. dioxane and tetrahydrofuran, and halogenated hydrocarbons, e.g. methylene chloride and chloroform.

In general the cyclization reaction is carried out at temperatures in the range from 20°C to 80°C usually from 40°C to about 60°C for a period of from 6 to 24 hours.

Suitable trivalent organophosphorus compounds are cyclic and/or acyclic trialkylphosphites, triarylphosphites and mixed aryl alkylphosphites or phosphoramides. The preferred trivalent organophosphorous compounds are a trialkylphosphites; the most preferred is triethylphosphite.

Generally, in the process of the invention, the carboxy group of the compounds [IVa, IVb), II and VI, as well as the starting penem of process A will be protected in the respective process. Thus, for instance, the alkylation reaction of process C will give a carboxy protected derivative of the compound of the invention which has subsequently to be deprotected. The hydroxy group of the hydroxyethyl attached to the 3-position of the azetidinone ring of the said compounds may also, conveniently, be protected.

Suitable hydroxy protecting groups Pg for use in the process of this invention are such groups conventionally used for such purpose in the β-lactam art and which are readily removable by procedures utilizing elemental zinc or any other conventional procedures. Preferred hydroxy protecting groups are trichloroethyloxycarbonyl, dimethyltertbutylsilyl, β-trimethylsilylethoxycarbonyl and trimethylsilyl; trimethylsilyl is a particularly preferred protecting group and is removable by treatment with a mild aqueous acid, such as aqueous acetic acid.

Suitable carboxy protecting groups are those conventionally used in the penem art and which can be removed under conventional conditions without reaction with other functional groups present on the penem molecule, for example allylic, p-nitrobenzyl and trichloroethyl. The preferred carboxy protecting group is allyl.

The removal of the protecting group from a protected carboxyl group can be carried out by conventional procedures selected according to the identity of the protecting group. For the removal of the preferred protecting group, allyl, this can in general be effected under catalytic conditions in the presence of a base, preferably by utilizing procedures described in our EP—A—0013663. THus an allyl group is preferably removed by utilizing a suitable aprotic solvent, such as tetrahydrofuran, diethyl ether or methylene chloride, with an alkali metal alkylcarboxylate, preferably potassium or sodium 2-ethylhexanoate (to give the alkali metal penem salt, preferably the sodium or potassium penem salt directly) or carboxylic acid, preferably 2-ethylhexanoic acid (to give the penem free-acid) and a mixture of a palladium compound and triphenyl phosphine as a catalyst.

Preparation of the foregoing salts and esters may be carried out according to conventional procedures, for forming salts of beta-lactams such as penicillins, cephalosporins and penems. Salts can be formed upon deprotection of an allyl group as above, or for example, by treating the free acid with metal compounds such as alkali metal salts of suitable carboxylic acids, or with ammonia or a suitable organic amine, wherein preferably stoichiometric amounts or only a samll-excess of the salt-forming agent is used. Acid addition salts of the compound can be obtained in the usual manner, for example by treating a compound of formula I with an acid or a suitable anion exchange reagent. The esters are preparable in a manner analogous to the preparation of the corresponding esters of penicillins and cephalosporins.

Salts may be converted in the usual manner into the free carboxy compounds, e.g. by treatment with tartaric acid, or into other salts, or pharmaceutically acceptable metabolizable esters.

The compounds of the invention may be produced in optically active form by utilizing optically active starting material in the reaction procedures.

The compounds of the invention may also be produced in mixtures with their stereoisomers, e.g. as racemic or diasteriomeric mixtures. For instance the 5R,6S,8R isomer may be produced with its enantiomer (mirror image) i.e. a 5S,6R,8S compound, in equal amounts when the starting compound is a racemic mixture. If desired, steroisomeric mixtures can be separated by standard techniques. For instance two enantiomers may be separated by fractional crystallization of optically active compounds, e.g. (−)-brucine, or (+)- and (−)-ephedrine salts.

Compound I for use in process A) can be readily prepared by the process described in EP—A-13662, that is by cyclising a compound

X

in which R is optionally protected hydroxyethyl, Pr and Pg are as defined above and Y is a phosphono group customary for a Wittig reaction, especially a triaryl, e.g. a triphenyl group. The reaction is generally conducted at a temperature of between 30° and 60°C and preferably at reflux temperatures in an organic solvent such as benzene, toluene or xylene under an inert atmosphere e.g. nitrogen or argen. Reaction time is e.g. 12—48 hours.

If R is protected hydroxyethyl, it will subsequently be deprotected before use in the process of the invention. By suitable selection of the protecting group it may be selectively deprotected, leaving group Pg in position protecting the 5-hydroxy group.

The sulfinyl compound II for use in process B can be readily prepared by treating a compound of the general formula XI

XI

in which $R_2$ is an organic group different from carbamoyloxyethyl, with a mild oxidizing agent e.g. m-chloroperoxybenzoic acid, in an inert solvent, for example dichloromethane at between −30°C and 20°C, e.g. at 0°C to 5°C. If desired the 3-carboxy group and 8-hydroxy group can be in protected form. Such compounds of formula XI can be prepared by methods generally known in the penem literature e.g. by the method disclosed in European Patent Application Publications Nos. 13662 and 58317.

The tautomeric compound of formula [IVa, IVb] can be prepared by the methods disclosed in EP—A—110280.

This procedure, in a preferred form, comprises:

a) recting an azetidinone of formula XX

XX

in which $R^3$ is a sulfur protecting group, e.g. triphenylmethyl, with an α-substituted alkylacetate of formula XXI

$$WCH_2\overset{O}{\overset{\|}{C}}—OCH_2CH=CH_2 \qquad XXI$$

in which W is a leaving group e.g. iodo or bromo to form a compound of formula XXII

XXII

This reaction can be carried out at 15° to 30°C in the presence of an acid acceptor. Preferably the reaction is conducted in acetonitrile employing cesium carbonate or tetraalkyl ammonium hydroxide as the acid acceptor;

b) treating the compound of formula XXII with a stoichliometric excess of elemental zinc in hydrochloric acid in an suitable organic solvent such as tetrahydrofuran at 15°C to 25°C, to form the compound of formula XXIII

XXIII

c) protecting the hydroxy group at the 5 position with a trimethylsilyl group by reaction of compound of formula XXIII with bis-trimethyl silylacetamide; the reaction suitably being carried out in a solvent such as dimethylformamide at 0°C to 30°C;

d) reacting the resulting protected compound with a thiocarbonyl compound of formula XXIV

$$S{=}C(Y)_2 \qquad \text{XXIV}$$

in which Y is a leaving group such as naphthyloxy or imidazolyl, generally using the same solvent as in the preceding step at a temperature in the range 10°C to 45°C to form the compound XXV

XXV

in which $P_g'$ is trimethylsilyl and Y is as defined above;

e) treating compound XXV with an equimolar amount of a strong base such as lithium diisopropyl amide, to produce a compound [IVa, IVb]; the reaction will generally be carried out in an anhydrous inert organic solvent, such as tetrahydrofuran, with the base being added to a solution of compound XXV in the solvent; typical reaction temperatures are −50°C to −100°C, and the reaction will generally be complete within 5 minutes to 24 hours.

As well as allyl, as a carboxy protecting group, other carboxy protecting groups known in the penem art can be used, e.g. p-nitrobenzyl.

The starting compounds VI of process D) are preferably prepared by the reaction of a compound of the formula XII

XII

with a reactive derivative e.g. chloride, of an acid of the formula XIII

```
              O
              ‖
      O       C
      ‖     /   \
Pr-O-C               OH
```

XIII

in which Pr is as defined above. This reaction will usually be carried out under normal acylating conditions, namely in an inert solvent e.g. methylene chloride and in the presence of a base e.g. an organic base such as a tertiary amine e.g. diisopropylethylamine, possibly together or with an inorganic base e.g. calcium carbonate.

Such process is described in more detail in the European Patent Application publication No. 58317.

Preparation A

Allyl-(5R,6S,8R)-2-(ethanesulfinyl)-6-(hydroxyethyl)-penem-3-carboxylate

Stir at 0°C to 5°C a solution of allyl-(5R,6S,8R)-2-ethylthio-6-(1-hydroxyethyl)penem-3-carboxylate (31.5 g) in ethyl acetate (200 ml) and dichloromethane (100 ml) and add over 30 minutes a solution of m-chloroperoxybenzoic acid (80—85%; 22 g) in ethyl acetate (120 ml). After a further 30 minutes, add the solution to a stirred mixture of ethyl acetate (150 ml), water (125 ml) and sodium bicarbonate (15 g), and stir rapidly for 15 minutes. Dry the organic phase over $MgSo_4$, evaporate and chromatograph rapidly on silica gel, elute using 1:1 hexane-ethyl acetate followed by pure ethyl acetate. Evaporate the product fractions and subject the residue to high vacuum to give the title compound as a thick yellow oil.

NMR ($CDCl_3$): δ 1.2—1.6 (m, 6H), 3.0—3.35 (m, 2H), 3.38 (br.s, 1H, exch by $D_2O$), 3.83 (m, 1H), 4.18 (m, 1H), 4,75 (br.d, J=6.5Hz), 5.2—5.6 (m, 2H), 5.73 and 5.89 (both d, J=1.5Hz, total 1H) and 5.8—6.2 (m, 1H).

Preparation B

A) Potassium carbamoyloxyethyltrithiocarbonate

Add a solution of potassium hydroxide (0.132 g) in ethanol (5 ml) to a solution of mercaptoethanol-carbamate (0.3 g) in ethanol and carbon disulphide (0.56 g) at 0°C to 5°C with stirring and under nitrogen. After stirring for 30 minutes add ether to the reaction solution, collect the resulting title compound as a yellow powder and dry it. Yield 0.52 g.

B) 3-(1-(2,2,2-trichloroethoxycarbonyloxy)ethyl)-4-(carbamoyloxyethylthiocarbonothioylthio)-aze-tidin-2-one

Dissolve 1 g potassium carbamoyloxyethyltrithiocarbonate from step A) in 20 ml of 90% ethanol containing 0.5 ml carbondisulfide and 0.8 ml triethylamine. Cool the solution to 0°C to 5°C and stir, under nitrogen. Add to the reaction mixture 1.2 g of 3-(1-(2,2,2-trichloroethoxycarbonyloxyethyl)-4-acetoxy-azetidin-2-one in 12 ml methylene chloride. Stir the reaction mixture for 10 minutes, then dilute with ethylacetate. Wash the solution with aqueous citric acid, then water, then dry over magnesium sulfate. Evaporate the dried extract, then chromatograph the resulting crude product on silica gel, eluting with ethylacetate:hexane (1:1) to give 1.02 g of the title compound as a yellow oil.

C) 1-(2-allyloxy-1,2-dioxo-ethyl)-3-(1-2,2,2-trichloroethoxycarbonyl)ethyl)-4-carbamoyloxyethylthio-carbonothioylthioazetidin-2-one

Cool to 0°C a solution of the title compound (0.45 g) from step B in methylene chloride (3 ml) and allyloxyalylchloride (0.14 ml) containing calcium carbonate (0.4 g) and stir under nitrogen while adding dropwise a solution of di-isopropylethylamine (0.19 ml) in methylene chloride (1 ml). After 10 minutes add 5 ml of ice water to the reaction solution with vigorous stirring and filter the mixture. Separate the filtrate, dry the orgaic layer over magnesium sulfate and dilute with ethanol-free chloroform. The resulting solution of the title compound can then be used directly for intramolecular cyclisation of the title compound as described below.

Example 1

Sodium (5R,6S,8R)-2-(2-carbamoyloxyethylthio)-6-(1-hydroxyethyl)-penem-3-carboxylate

A) Allyl-(5R,6S,8R)-2-(carbamoyloxyethylthio)-6-(1-[trichloroethoxycarbonyloxy]-ethyl)-penem-3-carboxylate.

Stir at 0° to 5°C a mixture of 0.80 g powdered potassium cyanate and 1.1 g of allyl-(5R,6S,8R)-6-(1-[tri-chloroethoxycarbonyloxy]-ethyl)-2-(2-hydroxyethylthio)-penem-3-carboxylate in 5 ml of $CH_2Cl_2$ and add dropwise 1.1 g trifluoroacetic acid. Stir the mixture at 0° to 5°C for 30 minutes, and then for 6 hours at 25°C. Work up in $CH_2Cl_2$-aqueous $NaHCO_3$, then dry, evaporate and chromatograph the organic phase on silica gel in ether —$CH_2Cl_2$ to yield the title compound as a yellow oil.

IR spectrum ($CH_2Cl_2$ solution) ν max 3500, 1795, 1750 and 1700 $cm^{-1}$. NMR ($CDCl_3$): δ 1.53 (d, 3, J=7), 3.22 (m, 2), 3.90 (dd, 1, J=1.5 and 8.5), 4.32 (d, 2, J=7), 4.79 (s, 2) 4.5—5.0 (m, 4), 5.05—5.55 (m, 2), 5.66 (d, 1, J=1.5) and 5.7—6.2 (m, 1).

B) Allyl-(5R,6S,8R)-(2-(2-carbamoyloxyethylthio)-6-(1-hydroxyethyl)-penem-3-carboxylate

Stir a mixture of 0.47 g of the product of step A, 0.45 g zinc dust, 0.6 ml acetic acid, 0.6 ml water and 6 ml tetrahydrofuran (THF) for two hours at 25°C. Work up in ethylacetate-$H_2O$, dry and evaporate the organic phase, then subject it to thin layer chromatography using a mixture of ether and dichloromethane containing 25% by volume ether to yield the title compound as a pale yellow foam.

IR spectrum ($CH_2Cl_2$) ν max 3400, 1790, 1710 and 1690 $cm^{-1}$; H NMR (DMSO—$d_6$): δ 1.13 (d, J=7), 3.20 (m, 2), 3.85 (m, 2), 4.14 (t, 2, J=7), 5.64 (m, 2), 5.1—5.5 (m, 2), 5.71 (d, 1, J=1.5), 5.7—6.2 (m, 1) and 6.55 (br.s 2, exch. by $D_2O$).

C) Stir under nitrogen at 25°C, a mixture of 1.2 g of the compound produced in step B, 0.55 g of sodium 2-ethylhexanoate, 0.12 g triphenylphosphine and 40 ml dry tetrahydrofuran. Add 0.12 g tetrakis-(triphenylphosphine)palladium, then stir for 2 hours and add 100 ml ether, centrifuge and wash with ethyl acetate. Dissolve the resulting salt in water and chromatograph on 10 g of reverse-phase C—18 silica, eluting with water. Combine pure fractions and lyophilize to obtain the title compound as a pale yellow powder.

IR (Nujol mull) ν max 3500, 1775, 1770 and 1650—1600 $cm^{-1}$.

Example 2

Sodium (5R,6S,8R)-2-(2-carbamoyloxyethylthio)-6-(1-hydroxyethyl)-penem-3-carboxylate

A) Allyl-(5R,6S,8R)-2-(2-carbamoyloxyethylthio)-6-(1-hydroxyethyl)-penem-3-carboxylate

Stir a mixture of 1.8 g of 2-(carbamoyloxy)-ethanethiol, 4.4 g of the product of the preparation A and 80 ml of dichloromethane for 1 hour at 0° to 5°C while adding 1.6 ml of diisopropylethylamine. Chromatograph the mixture on silica gel, eluting with increasing concentrations (5—15%) of acetone in $CH_2Cl_2$. Evaporate the pure fractions to yield the title compound as a pale yellow solid, identical to the product of Example 1, step B.

B) The product from Step A is then deprotected by the procedure of Example 1, step C to yield the title compound.

Example 3

Sodium (5R,6S,8R)-6-(1-hydroxyethyl)-2-(carbamoyloxyethylthio)-penem-3-carboxylate

A) Add to a flask 0.5 g of an equilibrium mixture of allyl (5R,6S,8R)-4-(1-hydroxyethyl) 2-thione-penam-3-carboxylate and allyl(5R,6S,8R)-6-(1-hydroxyethyl)-2-thiol-penem-3-carboxylate, 0.45 g 2-carbamoyloxyethyliodide, 10 ml acetonitrile, 5 ml water and 0.2 g sodium bicarbonate. Stir the mixture for 2 hours at room temperature to obtain 0.55 g of allyl-(5R,6S,8R)-3-(1-hydroxyethyl)-2-(2-carbamoyloxyethylthio)-penem-3-carboxylate. Work up in ethylacetate-$H_2O$, dry, evaporate the organic phase, then subject it to thin layer chromatography using a mixture of ether and dichloromethane containing 25% by volume ether.

B) Deprotect the product from step A) as described in Example 1, step C, to yield the title compound.

Example 4

Sodium (5R,6S,8R)-6-(1-hydroxyethyl)-2-(carbamoyloxyethylthio)-penem-3-carboxylate

A) Reflux the solution obtained in Preparation B) under stirring while adding a solution of triethylphosphite (0.4 ml) in chloroform (5 ml) over 2 hours. Continue to reflux for a further 2 hours and then evaporate the solvent under reduced pressure. Chromatograph the residual oil on silica gel, eluting with ethylacetate-hexane (1:4) to obtain 0.24 g of allyl-(5R,6S,8R)-3-(1-(2,2,2-trichloroethoxy carbonyloxy)ethyl)-2-(carbamoyloxyethylthio)-penem-3-carboxylate

IR: 5.62 μ

NMR: ($CDCl_3$): 1.5 (d, J=7 cps), 3.18 (dt, J=J=6,3cps), 3.90 (dd, J=J=7.5, 1.5cps), 4.25 (r, J=6cps), 4.85 (s), 5.65 (d, J=1.5cps).

B) Cool to 0°C a solution of the penem product from step A) (0.45 g) in tetrahydrofuran (5 ml) containing water (0.8 ml) and acetic acid (2 ml), under nitrogen and add zinc dust (1 g) in small lots to the solution while stirring. After stirring 1.5 hours filter the solution, evaporate the filtrate under reduced pressure and dissolve the residue in ethylacetate. Wash the resulting solution successively with water, aqueous sodium bicarbonate and brine, dry over magnesium sulphate and evaporate to dryness. Crystallize the residue from ethylacetate:hexane to give 0.273 g of allyl-(5R,6S,8R)-3-(1-hydroxyethyl)-2-(carbamoyloxyethylthio)-penem-3-carboxylate.

C) Treat the penem product from step B) according to the procedure of Example 1, step C, to obtain the title compound.

In the following Examples the Active Ingredient is sodium 5R,6S,8R-6-(1-hydroxyethyl)-2-(2-carbamoyloxyethylthio)-penem-3-carboxylate.

Example 5

| Injectable Powder: (per vial) | g/vial | g/vial | g/vial |
|---|---|---|---|
| Active Ingredient<br>Sterile Powder | 0.5 | 1.0 | 2.0 |

Add sterile water for injection or bacteriostatic water for injection, USP for reconstitution.

Example 6

| Injectable Solution | mg/ml | mg/ml |
|---|---|---|
| Active Ingredient | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture

1. Dissolve parabens in a portion (85% of the final volume) of the water for injection at 65° to 70°C).
2. Cool to 25° to 35°C charge and dissolve the sodium bisulfite, disodium edetate and sodium sulfate.
3. Charge and dissolve the Active Ingredient.
4. Bring the solution to final volume by adding water for injection.
5. Filter the solution through 0.22 micron membrane and fill into appropriate containers.
6. Terminally sterilize the units by autoclaving.

Example 7

Powder for preparation of injectable solution: (lyophilized powder, per vial)

| | g/vial | g/vial |
|---|---|---|
| Active Ingredient | 1.0 | 2.0 |
| Sodium Citrate | 0.05 | 0.10 |

Add sterile water for injection or bacteriostatic water for injection for reconstitution.

**Claims for the Contracting States: BE CH DE FR IT LI LU NL SE**

1. 5R,6S,8R-6-(1-hydroxyethyl)-2-(2-carbamoyloxyethylthio)penem-3-carboxylic acid and the pharmaceutically acceptable salts and metabolizable esters thereof.

2. An alkali metal salt of the compound named in claim 1.

3. A compound as defined in claim 2, characterized in that it is the sodium salt.

4. A pharmaceutical composition characterized by a compound as defined in any one of claims 1, 2 or 3, in admixture with a pharmaceutically acceptable carrier or excipient.

5. A process for preparing 5R,6S,8R-6-(1-hydroxyethyl)-2-(2-carbamoyloxyethylthio)penem-3-carboxylic acid and the pharmaceutically acceptable salts and metabolizable esters thereof, characterized by:

A) reaction of a compound of the general formula

I

wherein Pg is a hydroxy protecting group and Pr is a carboxy protecting group, with an alkali metal cyanate in the presence of trifluoroacetic acid or trichloroacetic acid;

B) reaction of a compound of the general formula

II

in which R is an organic radical different from carbamoyloxyethyl and any functional groups are protected if necessary or desired, with a compound of the general formula

$$HS\ CH_2CH_2O-\overset{O}{\overset{\|}{C}}-NH_2 \qquad \text{III}$$

or a reactive derivative thereof;

C) reaction of a compound of the general formula

IVa ⇌ IVb

in which any functional groups are protected if necessary.or desired, with a compound having the general formula

$$L-CH_2CH_2O-\overset{O}{\overset{\|}{C}}-NH_2 \qquad \text{V}$$

in which L is a leaving group,

D) reaction of a compound of the general formula

VI

in which any functional groups are protected if necessary or desired, with a trivalent organophosphorus compound

the process A, B, C or D being followed by one or more of the following operations, if necessary or desired;

(i) deprotection of one or more protected groups;
(ii) separation of the (5R,6S,8R) stereoisomer;
(iii) conversion of a free acid to a pharmaceutically acceptable salt or metabolisable ester;
(iv) conversion of a salt or ester to a free acid;
(v) transesterifying an ester or converting an ester into a pharmaceutically acceptable salt;
(vi) converting a salt into another salt or an ester.

**Claims for the Contracting State: AT**

1. A process for preparing 5R,6S,8R-6-(1-hydroxyethyl)-2-(2-carbamoyloxyethylthio)penem-3-carboxylic acid and the pharmaceutically acceptable salts and metabolizable esters thereof, characterized by:

A) reaction of a compound of the general formula

OPg; S; $SCH_2CH_2OH$; N; O; COOPr — I

wherein Pg is a hydroxy protecting group and Pr is a carboxy protecting group, with an alkali metal cyanate in the presence of trifluoroacetic acid or trichloroacetic acid;

B) reaction of a compound of the general formula

OH; S; O ↑ S R; N; O; COOH — II

in which R is an organic radical different from carbamoyloxyethyl and any functional groups are protected if necessary or desired, with a compound of the general formula

$$HS\ CH_2CH_2O{-}\overset{\overset{O}{\|}}{C}{-}NH_2 \qquad III$$

or a reactive derivative thereof;

C) reaction of a compound of the general formula

OH; S; SH; N; O; COOH — IVa ⇌ OH; S; =S; N; O; COOH — IVb

in which any functional groups are protected if necessary or desired, with a compound having the general formula

$$L{-}CH_2CH_2O{-}\overset{\overset{O}{\|}}{C}{-}NH_2 \qquad V$$

in which L is a leaving group,

D) reaction of a compound of the general formula

VI

in which any functional groups are protected if necessary or desired, with a trivalent organophosphorus compound

the process A, B, C or D being followed by one or more of the following operations, if necessary or desired;

(i) deprotection of one or more protected groups;

(ii) separation of the (5R,6S,8R) stereoisomer;

(iii) conversion of a free acid to a pharmaceutically acceptable salt or metabolisable ester;

(iv) conversion of a salt or ester to a free acid;

(v) transesterifying an ester or converting an ester into a pharmaceutically acceptable salt;

(vi) converting a salt into another salt or an ester.

2. A process as defined in claim 1, characterized in that an alkali metal salt of the compound named in claim 1 is produced.

3. A process as defined in claim 2, characterized in that the sodium salt is produced.

4. A process for preparing a pharmaceutical composition characterized in that 5R,6S,8R-6-(1-hydroxyethyl)-2-(2-carbamoyloxyethylthio)-penem-3 carboxylic acid or a pharmaceutically acceptable salt or ester thereof, is admixed with a pharmaceutically acceptable carrier or excipient.

5. A process as defined in claim 4, characterized in that an alkali metal salt of 5R,6S,8R-6-(1-hydroxyethyl)-2-(2-carbamoyloxyethylthio)penem-3-carboxylic acid is used.

6. A process as defined in claim 5, characterized in that the sodium salt is used as said alkali metal salt.

7. A process for preparing a pharmaceutical composition characterized in that 5R,6S,8R-6-(1-hydroxyethyl)-2-(2-carbamoyloxyethylthio)-penem-3-carboxylic acid or a pharmaceutically acceptable salt or ester thereof, as defined in claim 1, 2 or 3, prepared by the process of any one of claims 1 to 3, is admixed with a pharmaceutically acceptable carrier or excipient.

**Patentansprüche für die Vertragsstaaten: BE CH DE FR IT LI LU NL SE**

1. 5R,6S,8R-6-(1-Hydroxyethyl)-2-(2-carbamoyloxyethylthio)penem-3-carbonsäure und die pharmazeutisch annehmbaren Salze und metabilisierbaren Ester davon.

2. Alkalimetallsalz der in Anspruch 1 genannten Verbindung.

3. Verbindung nach Anspruch 2, dadurch gekennzeichnet, daß sie das Natriumsalz ist.

4. Pharmazeutische Zusammensetzung gekennzeichnet durch eine in einem der Ansprüche 1, 2 oder 3 definierten Verbindung in Mischung mit einem pharmazeutisch annehmbaren Träger oder Hilfsstoff.

5. Verfahren zur Herstellung von 5R,6S,8R-6-(1-Hydroxyethyl)-2-(2-carbamoyloxyethylthio)penem-3-carbonsäure und den pharmazeutisch annehmbaren Salzen und metabilisierbaren Estern davon gekennzeichnet durch:

A) Umsetzung einer Verbindung der allgemeinen Formel

I

worin Pg eine Hydroxyschutzgruppe und Pr eine Carboxyschutzgruppe ist, mit einem Alkalimetallcyanat in Gegenwart von Trifluoressigsäure oder Trichloressigsäure;

B) Umsetzung einer Verbindung der allgemeinen Formel

II

in welcher R ein von Carbamoyloxyethyl verschiedener organischer Rest ist und alle funktionellen Gruppen, falls notwendig oder erwünscht, geschützt sind, mit einer Verbindung der allgemeinen Formel

$$HS\ CH_2CH_2O{-}\overset{O}{\overset{\|}{C}}{-}NH_2 \qquad III$$

oder einem reaktiven Derivat davon;

C) Umsetzung einer Verbindung der allgemeinen Formel

IVa IVb

in welcher alle funktionellen Gruppen, falls notwendig oder erwünscht, geschützt sind, mit einer Verbindung der allgemeinen Formel

$$L{-}CH_2CH_2O{-}\overset{O}{\overset{\|}{C}}{-}NH_2 \qquad V$$

in welcher L eine Abgangsgruppe ist,

D) Umsetzung einer Verbindung der allgemeinen Formel

VI

in welcher alle funktionellen Gruppen, falls notwendig oder erwünscht, geschützt sind, mit einer dreiwertigen Organophosphorverbindung,

wobei dem Verfahren A, B, C oder D, falls notwendig oder erwünscht, einer oder mehrere der folgenden Verfahrensschritte folgen können:

(i) Abspaltung der Schutzgruppen von einer oder mehreren der geschützten Gruppen;

(ii) Abtrennung des (5R,6S,8R)-Stereoisomers;

(iii) Überführung einer freien Säure in ein pharmazeutisch annehmbares Salz oder in einen metabolisierbaren Ester;

(iv) Überführung eines Salzes oder Esters in eine freie Säure;

(v) Umesterung eines Esters oder Überführung eines Esters in ein pharmazeutische annehmbares Salz;

(vi) Überführung eines Salzes in ein anderes Salz oder einen Ester.

**Patentansprüche für den Vertragsstaat: AT**

1. Verfahren zur Herstellung von 5R,6S,8R-6-(1-Hydroxyethyl)-2-(2-carbamoyloxyethylthio)penem-3-carbonsäure und den pharmazeutisch annehmbaren Salze und metabolisierbaren Ester davon gekennzeichnet durch:

A) Umsetzung einer Verbindung der allgemeinen Formel

OPg ; S ; $SCH_2CH_2OH$ ; N ; O ; COOPr — I

worin Pg eine Hydroxyschutzgruppe und Pr eine Carboxyschutzgruppe ist, mit einem Alkalimetallcyanat in Gegenwart von Trifluoressigsäure oder Trichloressigsäure;

B) Umsetzung einer Verbindung der allgemeinen Formel

OH ; S ; O ← S R ; N ; O ; COOH — II

in welcher R ein von Carbamoyloxyethyl verschiedener organischer Rest ist und alle funktionellen Gruppen, falls notwendig oder erwünscht, geschützt sind, mit einer Verbindung der allgemeinen formel

$$HS\ CH_2CH_2O{-}\overset{\overset{O}{\|}}{C}{-}NH_2 \qquad III$$

C) Umsetzung einer Verbindung der allgemeinen Formel

OH ; S ; SH ; N ; O ; COOH — IVa ⇌ OH ; S ; =S ; N ; O ; COOH — IVb

in welcher alle funktionellen Gruppen, falls notwendig oder erwünscht, geschützt sind, mit einer Verbindung der allgemeinen Formel

$$L{-}CH_2CH_2O{-}\overset{\overset{O}{\|}}{C}{-}NH_2 \qquad V$$

in welcher L eine Abgangsgruppe ist,

D) Umsetzung einer Verbindung der allgemeinen Formel

$$\text{VI}$$

in welcher alle funktionellen Gruppen, falls notwendig oder erwünscht, geschützt sind, mit einer dreiwertigen Organophosphorverbindung,
wobei dem Verfahren A, B, C oder D, falls notwendig oder erwünscht, einer oder mehrere der folgenden Verfahrensschritte folgen können:

(i) Abspaltung der Schutzgruppen von einer oder mehreren der geschützten Gruppen;

(ii) Abtrennung des (5R,6S,8R)-Stereoisomers;

(iii) Überführung einer freien Säure in ein pharmazeutisch annehmbares Salz oder in einen metabolisierbaren Ester;

(iv) Überführung eines Salzes oder Esters in eine freie Säure;

(v) Umesterung eines Esters oder Überführung eines Esters in ein pharmazeutische annehmbares Salz;

(vi) Überführung eines Salzes in ein anderes Salz oder einen Ester.

2. Verfahren nach Anspruch 1, dadurch gekennzeichnet, daß ein Alkalimetallsalz der in Anspruch 1 genannten Verbindung hergestellt wird.

3. Verfahren nach Anspruch 2, dadurch gekennzeichnet, daß das Natriumsalz hergestellt wird.

4. Verfahren zur Herstellung einer pharmazeutischen Zusammensetzung, dadurch gekennzeichnet, daß 5R,6S,8R-6-(1-Hydroxyethyl)-2-(2-carbamoyloxyethylthio)penem-3-carbonsäure oder ein pharmazeutisch annehmbares Salz oder pharmazeutisch annehmbare Ester davon mit einem pharmazeutisch annehmbaren Träger oder Hilfsstoff gemischt wird.

5. Verfahren nach Anspruch 4, dadurch gekennzeichnet, daß ein Alkalimetallsalz von 5R,6S,8R-6-(1-Hydroxyethyl)-2-(2-carbamoyloxyethylthio)penem-3-carbonsäure verwendet wird.

6. Verfahren nach Anspruch 5, dadurch gekennzeichnet, daß das Natriumsalz als Alkalimetallzalz verwendet wird.

7. Verfahren zur Herstellung einer pharmazeutischen Zusammensetzung, dadurch gekennzeichnet, daß 5R,6S,8R-6-(1-Hydroxyethyl)-2-(2-carbamoyloxyethylthio)penem-3-carbonsäure oder ein pharmazeutisch annehmbares Salz oder pharmazeutisch annehmbarer Ester davon gemäß der Definition in Anspruch 1, 2 oder 3 und hergestellt gemäß dem Verfahren nach einem der Ansprüche 1 bis 3, mit einem pharmazeutisch annehmbaren Träger oder Hilfsstoff vermischt wird.

**Revendications pour les Etats contractants: BE CH LI DE FR IT LU NL SE**

1. Acide 5R,6S,8R-6-(1-hydroxyéthyl)-2-(2-carbamoyloxyéthylthio)pénem-3-carboxylique et sels et esters métabolisables pharmaceutiquement acceptables de celui-ci.

2. Un sel de métal alcalin du composé selon la revendication 1.

3. Composé tel que défini dans la revendication 2, caractérisé en ce que c'est le sel de sodium.

4. Composition pharmaceutique caractérisée par un composé tel que défini dans l'une quelconque des revendications 1, 2 ou 3, en mélange avec un excipient ou véhicule pharmaceutiquement acceptable.

5. Procédé d'obtention de l'acide 5R,6S,8R-6-(1-hydroxyéthyl)-2-(2-carbamoyloxyéthylthio)pénem-3-carboxylique et ses sels et esters métabolisables pharmaceutiquement acceptables, caractérisé par:

A) réaction d'un composé de formule générale

$$\text{I}$$

où Pg est un groupe protecteur d'hydroxy et Pr est un groupe protecteur de carboxy,
avec un cyanate de métal alcalin en présence d'acide trifluoroacétique ou d'acide trichloroacétique;

B) réaction d'un composé de formule générale

II

dans laquelle R est un radical organique différent de carbamoyloxyéthyle et où tout groupe fonctionnel est protégé si nécessaire ou souhaité, avec un composé de formule générale

$$HS\ CH_2CH_2O-\overset{\underset{\|}{O}}{C}-NH_2 \qquad III$$

ou un dérivé actif de celui-ci;

C) réaction d'un composé de formule générale

IVa ⇌ IVb

dans laquelle tout groupe fonctionnel est protégé si nécessaire ou souhaité, avec un composé ayant la formule générale

$$L-CH_2CH_2O-\overset{\underset{\|}{O}}{C}-NH_2 \qquad V$$

dans laquelle R est un groupe partant,

D) réaction d'un composé de formule générale

VI

dans laquelle tout groupe fonctionnel est protégé si nécessaire ou souhaité, avec un composé organophosphoreux trivalent,

le procédé A, B, C ou D étant suivi par une ou plusieurs des opérations suivantes, si nécessaire ou souhaité;

(i) déprotection d'un ou plusieurs protecteurs;

(ii) séparation du stéréoisomère (5R,6S,8R);

(iii) conversion d'un acide libre en un sel ou ester métabolisable pharmaceutiquement acceptable;

(iv) conversion d'un sel ou ester en un acide libre;

(v) transestérification d'un ester ou conversion d'un ester en un sel pharmaceutiquement acceptable;

(vi) conversion d'un sel en un autre sel ou un ester.

**Revendications pour l'Etat contractant: AT**

1. Procédé d'obtention de l'acide 5R,6S,8R-6-(1-hydroxyéthyl)-2-(2-carbamoyloxyéthylthio)pénem-3-carboxylique et ses sels et esters métabolisables pharmaceutiquement acceptables, caractérisé par:

A) réaction d'un composé de formule générale

I

dans laquelle Pg est un groupe protecterur d'hydroxy et Pr est un groupe protecteur de carboxy, avec un cyanate de métal alcalin en présence d'acide trifluoroacétique ou d'acide trichloroacétique;

B) réaction d'un composé de formule générale

II

dans laquelle R est un radical organique différent de carbamoyloxyéthyle et où tout groupe fonctionnel est protégé si nécessaire ou souhaité, avec un composé de formule générale

$$HS\ CH_2CH_2O{-}\overset{\overset{\displaystyle O}{\|}}{C}{-}NH_2 \qquad \text{III}$$

ou un dérivé réactif de celui-ci;

C) réaction d'un composé de formule générale

IVa IVb

dans laquelle tout groupe fonctionnel est protégé si nécessaire ou souhaité, avec un composé ayant la formule générale

$$L{-}CH_2CH_2O{-}\overset{\overset{\displaystyle O}{\|}}{C}{-}NH_2 \qquad \text{V}$$

dans laquelle R est un groupe partant,

D) réaction d'un composé de formule générale

$$\text{VI}$$

dans laquelle tout groupe fonctionnel est protégé si nécessaire ou souhaité, avec un composé organophosphoreux trivalent,
le procédé A, B, C ou D étant suivi par une ou plusieurs des opérations suivantes, si nécessaire ou souhaité;

(i) déprotection d'un ou plusieurs groupes protégés;
(ii) séparation du stéréoisomère (5R,6S,8R);
(iii) conversion d'un acide libre en un sel ou ester métabolisable pharmaceutiquement acceptable;
(iv) conversion d'un sel ou ester en un acide libre;
(v) transestérification d'un ester ou conversion d'un ester en un sel pharmaceutiquement acceptable;
(vi) conversion d'un sel en un autre sel ou un ester.

2. Procédé tel que défini dans la revendication 1, caractérisé en ce qu'un sel de métal alcalin du composé cité dans la revendication 1 est produit.

3. Procédé tel que défini dans la revendication 2, caractérisé en ce que le sel de sodium est produit.

4. Procédé d'obtention d'une composition pharmaceutique caractérisé en ce que l'acide 5R,6S,8R-6-(1-hydroxyéthyl)-2-(2-carbamoyloxyéthylthio)pénem-3-carboxylique ou un sel ou ester pharmaceutiquement acceptable de celui-ci, est mélangé avec un excipient ou véhicule pharmaceutiquement acceptable.

5. Procédé tel que défini dans la revendication 4, caractérisé en ce que l'on utilise un sel de métal alcalin de l'acide 5R,6S,8R-6-(1-hydroxyéthyl)-2-(2-carbamoyloxyéthylthio)pénem-3-carboxylique.

6. Procédé tel que défini dans la revendication 5, caractérisé en ce que l'on utilise un sel de sodium comme sel de métal alcalin.

7. Procédé d'obtention d'une composition pharmaceutique caractérisé en ce que l'acide 5R,6S,8R-6-(1-hydroxyéthyl)-2-(2-carbamoyloxyéthylthio)pénem-3-carboxylique ou un sel ou ester pharmaceutiquement acceptables de celui-ci, tel que défini dans la revendication 1, 2 ou 3, préparé par le procédé selon l'une des revendications 1 à 3, est mélangé avec un excipient ou véhicule pharmaceutiquement acceptable.